# EUROPEAN PATENT APPLICATION

(11) **EP 4 238 587 A1**
(43) Date of publication of application: **06.09.2023**
(21) Application number: 22160699.9
(22) Date of filing: 08.03.2022
(51) Int. Cl.: A61L 2/20, A47G 29/00, G06F 21/00

(54) **STERILIZATION CONTAINER AND RESPECTIVE METHOD OF OPERATION**

(30) Priority: 02.03.2022 PT 2022117832
(71) Applicant: DRT Rapid - Protótipos e Moldes Lda, 2415-314 Leiria (PT)
(72) Inventor: PAULO DUARTE, Valdemar Manuel, 2415-314 Leiria (PT); DE OLIVEIRA NEVES, Pedro Miguel, 2415-314 Leiria (PT)
(74) Representative: do Nascimento Gomes, Rui

(57) **Abstract**

It is an object of the present invention a sterilization container. This solution presented provides that articles placed inside the container can be sterilized in a safe way for the user, the removal of the disinfected article from the interior of the container being only enabled depending on certain operating parameters. For that purpose, the container is comprised by at least one access door, a sterilization unit and a control unit.

## Description

### FIELD OF THE INVENTION

The present invention is enclosed in the field of sterilization devices. More particularly, the present disclosure relates to mechanisms for providing sterilization of articles.

### PRIOR ART

The threat of dissemination of harmful infectious agents engenders insecurity and fear in broad sections of the general population. Such harmful infectious agents can be transmitted and distributed either via mail or the articles of daily use, such as smartphones, keys or other objects.

In a pandemic situation, such as the one currently experienced in the world, caused by the SARS-CoV-2 coronavirus and the COVID-19 disease, this has led to profound changes in the daily activities of people and companies. As a result, all civil society is now required to take additional precautions related to their daily activities and interpersonal relationships, with the maintenance of social distance and continuous hygiene care being essential. These additional precautions are fundamentally aimed at limiting, reducing and, if possible, eliminating the chains of contagion and spread of the pandemic.

Activities as simple as buying food and receiving postal mail and parcels at home or at work now require special attention due to the need to sterilize the articles received. Furthermore, the very use of personal protective equipment, such as a face mask, protective visor, gloves, shoe covers, suits, etc., requires the need for periodic sterilization if they are reusable. For this reason, it is essential to guarantee that each person, family and company has access to sterilization equipment that allows eliminating the presence of the virus and containing the spread of the pandemic.

Existing sterilization devices allow disinfestation of articles by ultraviolet radiation or by ozone, being effective in this task. However, they still have significant limitations related to the interaction with a user, which constitutes a disadvantage both in terms of security and in terms of enhancing their use.

This disclosure intends to present an improved solution in relation to the state of the art.

### SUMMARY OF THE INVENTION

An article sterilization container is thus object of the present disclosure. This solution thus provides that articles placed inside can be sterilized in a safe way for the user, the removal of the disinfected article from the interior of the container being only enabled depending on certain operating parameters.

To this end, the sterilization container of the present disclosure comprises:
- at least one access door, for introducing and removing an article from the interior of the container;
- a sterilization unit, adapted to carry out a sterilization process of an article inside the container; and
- a control unit, comprising:
   - an actuation module, configured to control the sterilization process based on operating parameters; and
   - a sensor module, configured to monitor operating parameters of the sterilization process.

In particular, and in an essential aspect of the container of the present disclosure, the actuation module of the control unit is configured to block or unlock the access door, i.e., prevent or allow the opening of the access door respectively, depending on at least an operating parameter monitored by the sensor module. This ensures that the access door of the sterilization container is not opened during the sterilization process, which could represent a dangerous situation for the user's health, in addition to compromising the efficiency of the sterilization process itself.

In another object of the present disclosure, a mailbox is presented that includes the developed sterilization container.

In yet another object of the present disclosure, it is described a sterilization system comprising a plurality of sterilization containers as described in the present disclosure.

Finally, it is also object of the present disclosure, a method for operating the developed sterilization container. Said method comprising the following steps:
- detecting at least one article inside the container and the closing of an access door of the container;
- blocking the access door;
- starting the article sterilization process;
- monitoring at least one operating parameter of the sterilization process;
- unblocking the access door of the container, after the end of the sterilization process, depending on at least one operating parameter being monitored.

### DESCRIPTION OF FIGURES

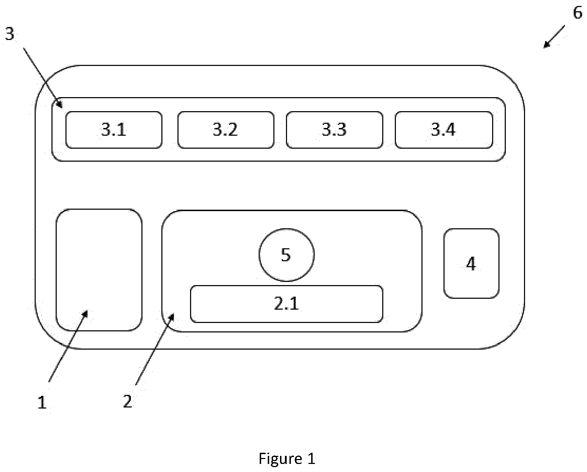

Figure 1 - block diagram representation of an embodiment of the sterilization container of the present disclosure. Reference numbers represent:
1 - access door;
2 - sterilization unit;
   2.1-ozone production module;
3 - control unit;
   3.1 - actuation module;
   3.2 - sensor module;
   3.3 - communication module;
   3.4 - user interface module;
4 - ventilation unit;
5 - article;
6 - sterilization container.

### DETAILED DESCRIPTION

The more general and advantageous configurations of the present invention are described in the Summary of the invention. Such configurations are detailed below in accordance with other advantageous and/or preferred embodiments of implementation of the present invention.

The present disclosure relates to an article sterilization container (6). The container (6) can be dimensioned according to the needs of a particular case, and in order to receive inside articles (5) of any shape to be sterilized. Particularly, in the context of the present disclosure an article can be a mail letter or parcel or can be a smartphone, a face mask, a protective visor, gloves, shoe covers, suits, etc.

Consequently, the at least one access door (1) has a shape/size such as to allow, once opened, the introduction of the article (5) to be sterilized inside the container (6) and its removal after the process has been completed. The access door (1) can be used for both the introduction and removal of articles (5) from inside the container (6). However, in an alternative scenario, the container (6) could be comprised of at least one access door (1) exclusively intended for introducing articles (5) into the container (6), and at least one access door (1) exclusively intended for removing articles (5) from the interior of the container (6). As an example of realization, and for articles (5) with dimensions up to 340x260x230 mm, the container (6) can be made of a Corian type material, with dimensions 1400x460x445 mm. In this example, the container (6) may have three access doors (1): a first access door for introducing thin articles, such as letters, with a size of 420x40 mm, a second access door for introducing more bulky articles, with a size of 435x400 mm and a third access door for removing articles (5) from the interior of the container, with a size of 435x605 mm. In view of the dimensions of such a container (6) it has the advantage of being portable and easily transported and installed in different locations.

In an advantageous aspect of the developed sterilization container (6), it comprises a mechanical system based on bars and hinges, which presents a geometry such that it is impossible to remove an article (5) that is inside the container (6) when the access door (1) is opened.

The control unit (3) comprises an actuation module (3.1) whose function is to control the sterilization process carried out by the sterilization unit (2). By controlling the sterilization process, it must be understood as all actions that allow programming the start, interruption or conclusion of the sterilization process. Additionally, the actuation module (3.1) of the control unit (3) is also configured to act in the blocking or unblocking of the access door (1) depending on the operating parameters that are being monitored by the sensor module (3.2). In particular, blocking the access door (1) prevents it from being opened at least while the sterilization process is taking place or while safety conditions are not guaranteed - by monitoring at least one operating parameter. Unlocking the access door (1) allows it to be opened by the user in order to safely remove the sterilized article (5) from inside the container.

In a preferred embodiment of the present disclosure, the sterilization unit (2) of the developed container (6) comprises an ozone production module (2.1). In an advantageous aspect of the present disclosure, the ozone production module (2.1) is located in a lower sub-compartment, inside the container (6), separated from the remaining interior space of the container (6), where the article (5) to be sterilized is placed, by means of a perforated plate, in a manner to allow the circulation of ozone inside the container (6). The module (2.1) operates through the electrolysis of air, which breaks down oxygen molecules into oxygen free radicals. These oxygen atoms join with other oxygen molecules, forming ozone. In order to monitor the ozone concentration throughout the sterilization process, and above all after this process has been completed, the sensor module (3.2) comprises at least one ozone concentration sensor.

In another preferred embodiment of the present disclosure, the sensor module (3.2) of the control unit (3) comprises at least one distance sensor, configured to detect the presence of at least one article (5) inside the container (6) from the determination of the empty space remaining inside the container (6). In an alternative embodiment the sensor module (3.2) comprises two distance sensors. Advantageously, the use of two distance sensors allows to gather sensory information about the dimensions of the articles (5) inside the container (6), as well as to monitor the status of the access door (1), in particular if it is closed or open. In fact, the sterilization process is only initiated by the sterilization unit (2) when an access door (1) is closed, in order to guarantee the necessary safety conditions for the user, in the space surrounding the container (6).

In another preferred embodiment of the present disclosure, the operating parameters related to the sterilization process relate to the presence of an article (5) within the container (6) and the concentration of ozone within the container (6). In this way, it is guaranteed that the sterilization process does not start without at least one article (5) being placed inside the container (6). In particular, the actuation module (3.1) of the control unit (3) is configured to block the access door (1) and trigger the sterilization unit (2) to carry out the sterilization process, if the presence of an article (5) inside the container (6) is detected. In addition, the actuation module (3.1) is also configured to unblock the access door (1) when the ozone concentration is below a pre-set value. Optionally the pre-set ozone concentration value is 0.2 ppm.

In another preferred configuration of the sterilization container (6) of the present disclosure, it comprises a ventilation unit (4) configured to:
recirculate the air inside the container (6) during the sterilization process; and to
extract air from inside the container (6) after completion of the sterilization process.

Thus, and considering that the ozone production module (2.1) is adapted to produces ozone from the oxygen present inside the container (6), the recirculation of the air inside it allows a greater dispersion of ozone, increasing the efficiency of the sterilization process. In addition, and after the sterilization process is completed, the extraction of the air inside the sterilization container (6) reduces the ozone concentration levels more quickly, allowing the user to collect the sterilized article (5) more quickly. In a particular embodiment of the sterilization container (6), the ventilation unit (4) comprises at least one fan for recirculating air inside the container (6) and at least one fan for extracting air inside the container (6). Optionally, said fans can be of 24V.

In another preferred embodiment of the present disclosure, the control unit (3) comprises a user interface module (3.4). Said module (3.4) comprises means adapted to implement a user authentication protocol, which may be based on one or a combination of the following authentication mechanisms: entry of an access code and/or reading of a QR code. Said means can be any one already known from the state of the art, in particular, the entry of the access code can be done through a touch screen type interface, or through proximity using Bluetooth or NFC technology. In an alternative embodiment, the user interface module (3.4) comprises a monitor configured to display at least information related to the blocking state of the access door (1), i.e., whether it is blocked or unblocked, or with the operating parameters, namely, the presence of an article (5) inside the container (6), the concentration of ozone inside the container (6), and the status of the sterilization process, that is, for example, the process phase, the elapsed time or the time that is still needed until complete the process. Additionally, the monitor can be used to present any other type of information related to the sterilization container (6), any of its constituent units and modules, or to the articles (5) place inside. For example, in a scenario where the container (6) of the present disclosure is integrated in a mailbox, the monitor can be used to display the address, house number, box serial number, condominium number or other type of identification, with which the mailbox is associated.

In a preferred embodiment, the actuation module (3.1) of the control unit (3) is programmed to unblock the access door (1) after the user interface module (3.4) authenticates a user. In particular, the opening the access door (1) can be enabled if the user interface module (3.4) recognizes the user and validates their authentication process. In this way, it is guaranteed that only users who are authenticated before the sterilization container (6) have access to the article (5) inside.

In a preferred embodiment of the present disclosure, the sterilization container (6) further comprises a unique identifier. Said unique identifier is associated with at least one user, whereby the user authentication process implemented by the interface module (3.4) becomes more robust since only the authentication of a user is validated, allowing him access to the interior of the container (6), if it is associated with the unique identifier of the respective container (6).

In another preferred embodiment of the present disclosure, the control unit (3) comprises a communication module (3.3), intended to provide the **container's** interaction with the outside world. The communication module (3.3) can be any one known from the state of the art. In turn, the user interface module (3.4) additionally comprises a user interface platform that is adapted to provide a user with remote control of the sterilization process, by programming the start of the sterilization process and providing data concerning the operating parameters and the blocking state of the access door (1), i.e., whether it is blocked or unblocked. Any type of information related to the sterilization container (6), its constituent units and modules, or related to the status of the sterilization process can be made available and accessed remotely by a user. This is possible through the communication module (3.3) of the control unit (3) and through a mobile device application, running for example on the user's smartphone, which is configured to communicate with the user interface platform through a wide area network, local area network or personal area network. All types of control and access to information can thus be carried out remotely, such as remotely programming a sterilization process for a letter/parcel/article that has been placed inside the container (6).

In another preferred embodiment of the present disclosure, the access door (1) comprises a magnetic clasp operable by the actuation module (3.1) of the control unit (3) and adapted to block or unblock the access door. In this way, it is possible to prevent the container (6) from being opened while the sterilization process is in progress or by an unauthorized person trying to open the access door (1). In an advantageous aspect of the container (6), the magnetic clasp is an electromagnet. In an alternative embodiment, the access door (1) comprises an additional access mechanism to enable opening of the access door (1) when in the unblocked state. For example, said additional mechanism could be implemented by any mechanism already known in the state of the art, for example through a lock for which a corresponding key will be needed, or through the introduction of an access code via a touch screen or proximity interface, via Bluetooth or NFC, in which case the user interface module (3.4) of the control unit (3) can be used. Alternatively, said mechanism could be an operating command issued via the user interface platform, through the mobile device application. This ensures that the access door (1) is only opened by someone who has access to this additional security mechanism.

It is also object of the present disclosure, a sterilization system comprising a plurality of sterilization containers (6) as presented herein. In a preferred embodiment of the system, it additionally comprises at least one sterilization container registration database and a management module, in which these two elements and the plurality of sterilization containers (6) are connected via a communication network. More particularly, the management module comprises an interface platform adapted to provide a user's access to the sterilization container registration database and the user's association with at least one sterilization container (6), through a mobile device application configured to communicate with the interface platform through a wide area network, local area network or personal area network. In this way, having access to the list of all existing sterilization containers (6), for example within a certain geographical area, it is possible for the user to reserve a sterilization container (6) and use it to sterilize an article (5). For this purpose, additional information will have to be shared and processed in order to carry out a screening to allow the booking action only to those sterilization containers (6) that are available, that is, for example, without a user associated with their unique identifier or with no articles (5) inside.

It is also object of the present disclosure a method for operating the presented sterilization container (6). In a preferred embodiment of the method, the sterilization process can only be started after closing the access door (1) and if the presence of an article (5) inside the container (6) is detected. The sensor module (3.2) is adapted to carry out this verification.

In a preferred embodiment of the method, the operating parameter is the ozone concentration, and in which the sterilization process is carried out for a predefined period of time or until the ozone concentration reaches a predefined value, optionally that value being equal to 5ppm. Upon completion of the sterilization process the ozone concentration is monitored and the access port (1) unblocking step is performed only when the ozone concentration levels are below a pre-set value, for example 0.2ppm. In a preferred embodiment of the method, and taking into account the ability of the sterilization container (6) to communicate with the outside world, it also includes the steps of notifying the user of the end of the sterilization process, when it is safe to open the access door (1), and notifying the user of the opening of the access door (1).

In a preferred embodiment of the method, during the sterilization process, the ventilation unit (4) is activated to recirculate the air inside the container (6). Optionally, at least one air recirculation fan is activated. After the sterilization process is completed, the ventilation unit (4) is activated to extract the air inside the container (6). Optionally, at least one air extraction fan inside the container (6) is activated.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. A sterilization container (6) for articles comprising:
- at least one access door (1), for introducing and removing an article (5) from the interior of the container (6);
- a sterilization unit (2), adapted to carry out a sterilization process of an article (5) inside the container (6);
- a control unit (3), comprising:
- an actuation module (3.1), configured to control the sterilization process based on operating parameters;
- a sensor module (3.2), configured to monitor operating parameters of the sterilization process;
wherein,
the actuation module (3.1) of the control unit (3) is configured to block or unblock the access door (1), i.e., prevent or allow opening of the access door (1) respectively, as a function of at least one operating parameter monitored by the sensor module (3.2).

2. Container (6) according to claim 1, wherein the sterilization unit (2) comprises an ozone production module (2.1); and
Wherein,
the sensor module (3.2) comprises at least one ozone concentration sensor.

3. Container (6) according to any of the preceding claims, wherein the sensor module (3.2) comprises at least one distance sensor configured to detect the presence of at least one article (5) inside the container (6), based on the determination of the space remaining empty within the container; optionally, the sensor module (3.2) comprises two distance sensors.

4. Container (6) according to claims 2 and 3, wherein the operating parameters of the sterilization process are: presence of an article (5) inside the container (6) and concentration of ozone inside the container (6);
Wherein,
the actuation module (3.1) of the control unit (3) is configured to:
block the access door (1) and activate the sterilization unit (2) to carry out the sterilization process, if the presence of an article (5) inside the container (6) is detected; and to
unblock the access door (1) when the ozone concentration is below a pre-set value; optionally the pre-set ozone concentration value is 0.2 ppm.

5. Container (6) according to any of the preceding claims, further comprising a ventilation unit (4) configured to:
- recirculate the air inside the container (6) during the sterilization process; and to
- extract air from inside the container (6) after completion of the sterilization process;
optionally, the ventilation unit (4) comprises:
- at least one fan adapted to recirculate air inside the container (6); and
- at least one fan adapted to extraction air from inside the container (6).

6. Container (6) according to any of the preceding claims, wherein the control unit (3) comprises a user interface module (3.4); said module (3.4) comprising:
means adapted to implement a user authentication protocol based on one or a combination of the following authentication mechanisms: entry of access code and/or reading of a QR code;
optionally, the introduction of the access code is done through a touch screen type interface; and
optionally, the entry of the access code is done by proximity via Bluetooth or NFC;
optionally, the user interface module (3.4) comprises a monitor configured to display at least information related to: access door blocking status; and operating parameters such as presence of an article (5) within the container (6), concentration of ozone within the container (6), and status of the sterilization process.

7. Container (6) according to claim 6, wherein the actuation module (3.1) of the control unit (3) is programmed to unblock the access door (1) after the user interface module authenticates a user.

8. Container (6) according to claim 7, further comprising a unique identifier; said unique identifier being associated with at least one user;
wherein,
the user interface module (3.4) is configured to:
authenticate at least one user associated with the **container's** unique identifier;
do not authenticate a user not associated with the container's unique identifier.

9. Container (6) according to any of claims 6 to 8, wherein the control unit (3) comprises a communication module (3.3);
wherein,
the user interface module (3.4) further comprises a user interface platform adapted to provide remote control of the sterilization process by programming the start of the sterilization process and information about the operating parameters and the blocking status of the access door (1) through the communication module (3.3) of the control unit (3), by means of a mobile device application configured to communicate with the user interface platform through a wide area network, local area network or personal area network.

10. Container (6) according to any of the preceding claims, wherein the access door (1) comprises a magnetic clasp operable by the actuation module (3.1) of the control unit (3) and adapted to block or unblock the access door (1);
optionally, according to claims 6 and 9, the access door (1) comprises an additional access mechanism to enable opening of the access door (1) when it is in the unlocked state; said additional mechanism being one or a combination of the following mechanisms: door lock; entry of an access code via a touch screen or proximity interface via Bluetooth or NFC using the control unit's user interface module (3.4); operating command issued via the user interface platform.

11. A mailbox comprising the sterilization container according to any of the claims 1 to 10.

12. Sterilization system comprising:
- a plurality of sterilization containers (6) according to any of the claims 8 to 10;
- a sterilization container registration database; and
- a management module;
interconnected through a communication network;
wherein,
the management module comprises an interface platform adapted to provide a user's access to the sterilization container registration database and the user's association with at least one sterilization container (6), through a mobile device application configured to communicate with the interface platform through a wide area network, local area network or personal area network.

13. A method of operating the sterilization container (6) of the claims 1 to 10; the method comprising the following steps:
- detecting at least one article (5) inside the container (6) and the closing of an access door (1) of the container (6);
- blocking the access door (1);
- starting the article sterilization process;
- monitoring at least one operating parameter of the sterilization process;
- unblocking the access door (1) of the container, after the end of the sterilization process, depending on at least one operating parameter being monitored.

14. The method according to claim 13, wherein an operating parameter is ozone concentration; and in which the sterilization process is performed
during a pre-set period of time; or
until the ozone concentration reaches a pre-set value, optionally this value being equal to 5ppm;
and
after completion of the sterilization process the ozone concentration is monitored; and
the unblocking step of the access door (1) is performed when the ozone concentration levels are below a pre-set value; optionally the pre-set ozone concentration value is 0.2 ppm;
optionally,
the method including the steps of:
- notifying the user of the end of the sterilization process; and
- notifying the user of the opening of the access door (1).

15. The method according to claims 13 or 14, wherein
during the sterilization process, a ventilation unit (4) is activated to recirculate the air inside the container (6); optionally at least one air recirculation fan is activated; and
after the sterilization process is completed, the ventilation unit (4) is activated to extract the air inside the container (6); optionally, at least one air extraction fan is activated.
